# EUROPEAN PATENT APPLICATION

(11) **EP 1 371 964 A1**
(43) Date of publication of application: **17.12.2003**
(21) Application number: 02729568.2
(22) Date of filing: 15.01.2002
(51) Int. Cl.: G01N 1/04, G01N 33/48, G01N 33/50

(54) **DEVICE AND METHOD FOR SAMPLING FECES**

(30) Priority: 16.01.2001 JP 2001007742
(71) Applicant: International Reagents Corporation, Kobe-shi Hyogo 651-2271 (JP)
(72) Inventor: IORI, Hitomi c/o INTERNATIONAL REAGENTS CORP., Kobe-shi, Hyogo 651-2271 (JP); KAJIWARA, Nobuya c/o INTERNATIONAL REAGENTS CORP., Kobe-shi, Hyogo 651-0083 (JP); OCHIAI, Koji c/o INTERNATIONAL REAGENTS CORP., Kobe-shi, Hyogo 651-22 (JP)
(74) Representative: Forstmeyer, Dietmar, Dr. rer. nat., Dipl.-Chem.
(86) International application number: JP0200173
(87) International publication number: WO02055994

(57) **Abstract**

An implement and a device for sampling feces specimen designed to be able to sample the specimen by a specified amount and having a function to prevent the specimen from being sampled a plurality of times and a function to prevent the sampling implement once stored in a feces sampling container from being moved reversely or extracted; the feces sampling implement comprising a bar or stick portion having projected parts and/or recessed parts in the parts thereof, whereby the feces sampling implement cannot be moved reversely or extracted or becomes hard to be moved reversely or extracted after once inserted into the feces sampling container for storage, and thus an extraction prevention function can be developed.

## Description

### Technical Field

The present invention relates generally to a device and method for collecting samples used in clinical tests, and more particularly to an implement for collecting feces (feces sampling implement), a feces sampling container, a feces sampling device, a feces sampling method, a method for preparing samples of occult blood in feces, and a method of testing for occult blood in feces.

### Background Art

Testing for occult blood in feces is widely conducted as a screening test for colon cancer. In contrast to ordinary cl inical tests, methods for collecting test samples in such testing have not been made specific. Because the subject collects the test sample, feces sampling containers that are variously devised so that it is possible to sample a specific amount of feces have been developed. Numerous containers have been devised, such as containers that enable a specific amount of feces to be sampled by passing a stick-with which a sample has been collected-through a funnel-shaped partition portion, containers where a tip end portion of a feces sampling stick is made into a brush form or a spiral shape, and containers that use a porous member.

In such conventional technology, feces sampling devices having various shapes and functions have been developed. However, because the amount of sampled feces is usually about 1 mg to 50 mg, which is a very small amount, sometimes the subject feels uneasy about the paucity of the amount of sampled feces and repeatedly samples the feces a second or third time after the feces has been sampled. With such sampling, precise determination becomes impossible, which leads to erroneous diagnosis.

It is an object of the present invention to provide, with respect to a device for collecting a feces sample, a feces sampling implement, a feces sampling container and a feces sampling device designed so that a regular amount of a sample can be collected. It is also an object of the invention to provide a feces sampling implement, a feces sampling container and a feces sampling device disposed with a function for preventing multiple collections of a sample by desorption.

### Disclosure of the Invention

As a result of their extensive research, the present inventors devised a device that, with respect to a device that collects biological samples and particularly feces, physically prevents multiple sample collection by providing a feces sampling device having a function for preventing a feces sampling implement once accommodated in a feces sampling container from being reversely moved or pulled out, and thus completed the present invention.

That is, the present invention provides:
1. In a feces sampling device including a feces sampling implement and a feces sampling container that can accommodate the feces sampling implement, a feces sampling implement comprising a measure that can control further taking out and putting in of the feces sampling implement once the feces sampling implement has been inserted and accommodated inside the feces sampling container;
2. The feces sampling implement of item 1, wherein a reverse-motion or pullout prevention function measure is effected after the feces sampling implement has been inserted and accommodated in the feces sampling container;
3. The feces sampling implement of item 2, wherein the reverse-motion or pullout prevention function is retained by the feces sampling implement including convex portions and/or concave portions at a part of its structure;
4. A feces sampling device retaining a reverse-motion or pullout prevention function by combining a container that can accommodate the feces sampling implement of at least any one of items 1 to 3 and the feces sampling implement;
5. The feces sampling device of item 4, wherein a funnel-shaped member is disposed at a part of the feces sampling container that can accommodate the feces sampling implement;
6. The feces sampling device of item 4 or 5, wherein a thin film is disposed at an insertion slot for the feces sampling implement in the feces sampling container that can accommodate the feces sampling implement;
7. A feces sampling method using the feces sampling implement or the feces sampling device of at least any one of items 1 to 6;
8. A method for preparing a sample of occult blood in feces by the feces sampling method of item 7; and
9 . A method of testing for occult blood in feces using the sample obtained in item 8.

### Brief Description of Drawings

Fig. 1 shows a feces sampling implement disposed with a convex reverse-motion prevention portion (Experimental Example 1);
Fig. 2 shows a state where the feces sampling implement disposed with the convex reverse-motion prevention portion is inserted into a feces sampling container and fitted together with a funnel-shaped member inside the container (Experimental Example 1) ;
Fig. 3 shows a state where a feces sampling implement disposed with a concave reverse-motion prevention portion is inserted into a feces sampling container and fitted together with a funnel-shaped member inside the container (Experimental Example 2);
Fig. 4 shows a feces sampling container disposed with a thin film at a feces sampling implement insertion slot (Experimental Example 3);
Fig. 5 shows a state where a feces sampling implement is inserted and accommodated in the feces sampling container disposed with the thin film (Experimental Example 3); and
Fig. 6 shows a state where the feces sampling implement accommodated in the feces sampling container disposed with the thin film is being pulled out (Experimental Example 3).

### Explanation of Reference Numerals

1 Liquid inside feces sampling container
2 Feces sampling implement insertion slot of feces sampling container
3 Reverse-motion prevention portion in feces sampling device
4 Tip end portion of feces sampling implement

### Best Mode for Carrying Out the Invention

The feces sampling implement of the invention is an implement used when collecting feces used in feces testing and in which multiple use for feces sampling is controlled. This control is the result of a design with respect to which, for example, physically controlled processing has been effected. By multiple use being physically controlled is meant that, once the feces sampling implement has been inserted inside a feces sampling container that accommodates the feces sampling implement after feces sampling (referred to below simply as a "feces sampling container") and a feces sample is accommodated, multiple use is controlled by making it impossible for the feces sampling implement to be removed again or by making it difficult for the feces sampling implement to be taken out and put in again. Thus, the object of the invention can be achieved. In addition, various well-known means, such as a warning resulting from changes in color or shape and also movement control resulting from shape changes can be adopted as control means.

One example of physically controlled specific means is a feces sampling implement that includes a stick-shaped region, with convex portions and/or concave portions being disposed at a part thereof. By disposing convex portions and/or concave portions, it becomes impossible for the feces sampling implement to be reversely moved or pulled out once the feces sampling implement has been inserted and accommodated in the feces sampling container, and a pullout prevention function is exhibited. Moreover, in order to control movement of the feces sampling implement, a pullout prevention function and a feces scrape-off function can also be combined with convex portions and/or concave portions of a feces scrape-off portion include data tip end portion of the feces sampling implement.

The present invention provides a feces sampling device having the function of preventing the feces sampling implement frombeing reversely moved or pulled out, by combining the above-described feces sampling implement with a feces sampling container shape, whereby an effective and reliable feces sampling method can be achieved and implemented by using the feces sampling implement and/or the feces sampling device.

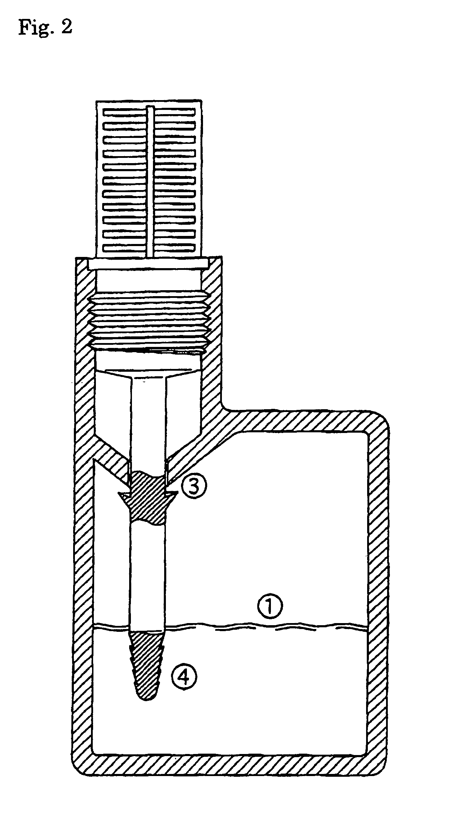

For example, by combining a funnel-shaped member disposed at a part of the feces sampling container with the above-described control means of the feces sampling implement, the amount of sampled feces can be made specific and a feces sampling device having the function of preventing the feces sampling implement from being reversely moved or pulled out can be achieved. Here, a funnel-shaped member that has the function of scraping off excessive feces when the feces sampling implement is inserted and accommodated in the feces sampling container is usually used. As one example, there is a funnel-shaped bulge inside the feces sampling container of Fig. 2. Although a funnel shape is preferable, the shape of the member is not particularly limited. It suffices as long as the member at least includes the function of scraping off excessive feces and the function of being able to prevent the inserted and accommodated feces sampling implement from being reversely moved or pulled out. As another mode, the feces sampling implement may be inserted into a feces sampling container where an insertion slot for the feces sampling implement in the feces sampling container is closed off by a thin film, so that the thin film is broken when the feces sampling implement is inserted therethrough. In this case, the thin film faces the inside of the container body as it is retained in the feces sampling container. By combining the thin film facing inward with the feces sampling implement including the control means such as the convex portions and/or the concave portions, it is possible to impart the function of preventing the feces sampling implement from being reversely moved or pulled out. With respect to the thickness of the thin film, it is necessary for the thin film to be strong enough so that there is no liquid leakage and so that the thin film can be pierced by a feces sampling stick. More preferably, it is preferable for the thin film to be strong enough to prevent the feces sampling implement from being pulled out.

As for the combination of the funnel-shaped member and the feces sampling implement, a material that is elastic or plastic is used for the funnel-shaped member and a material that is more rigid than the funnel-shaped member is chosen for the material of the feces sampling implement, whereby it is possible to achieve a well-fitting combination.

The feces sampling implement and the container that can accommodate the feces sampling implement of the invention can be produced as molded products of ordinary synthetic resin. Although the materials are not particularly limited, polyethylene, polypropylene, polystyrene and ABS resins are usable.

In this manner, the invention prepares samples for testing for occult blood in feces by using the feces sampling implement and the feces sampling device having the above-described functions, and enables reliable testing for occult blood in feces with these samples. Although the sample for testing for occult blood in feces is prepared from the feces accommodated inside the feces sampling container, the method of sample preparation is selected in accordance with testing content. Testing for occult blood in feces can be conducted according to well-known methods or according to methods yet to be developed. Specific examples include a chemical testing method and an immunological testing method. The chemical testing method is a method that determines the peroxidase-like activity of hemoglobin by oxidative coloring of a chromogen. In this case, test paper, in which filter paper is soaked in a chromogen, is coated with feces and a hydrogen peroxide solution acting as a coloring liquid is dropped onto the coated feces to determine the presence or absence of coloring. Examples of the immunological measurement method include immuno latex agglutination, hemagglutination or immunochromatography. Although collection of the test sample from the inside of the feces sampling container is usually conducted by closing in a part (e.g., shoulder portion, bottomportion, side portion, etc.) of the feces sampling container or adhering a seal member on the part (e.g., shoulder portion, bottom portion, side portion, etc.) and sticking a nozzle into this portion and absorbing, collection is not limited to this.

### Experimental Examples

The invention will be further described below using the drawings as experimental examples. However, the invention is not limited to the experimental examples.

### Experimental Example 1

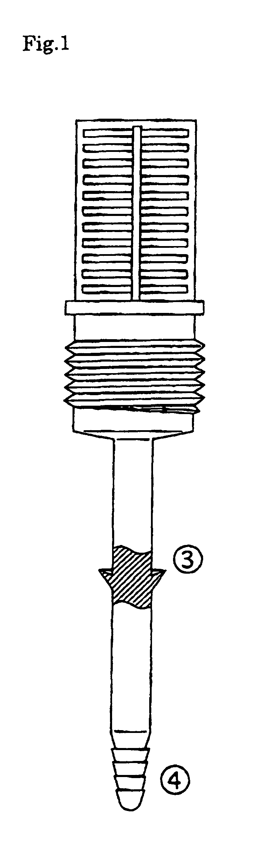

Fig. 1 is an example where a convex reverse-mot ion prevention device is included at a part of a feces sampling implement. When the feces sampling implement is accommodated in a feces sampling container after a subject has sampled feces with the feces sampling implement, the feces sampling implement is passed through a funnel-shaped sample scrape-off portion disposed inside the container, whereby excessive feces is scraped off and a specific amount of the sample can be collected inside the container. A reverse-motion prevention portion disposed at a part of a stick of the accommodated feces sampling implement is fitted together with the funnel-shaped scrape-off portion (Fig. 2) . Once the feces sampling implement is accommodated, the feces sampling implement cannot be pulled out or it becomes difficult for the feces sampling implement to be pulled out. Thus, multiple feces sampling can be prevented.

### Experimental Example 2

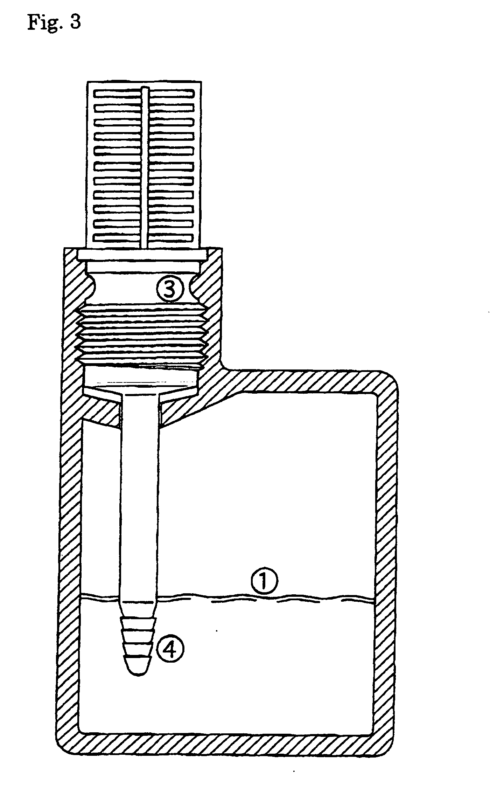

Fig. 3 is an example where the reverse-motion prevention portion of the feces sampling implement is concave. The function of this reverse-motion prevention portion is the same as that of the convex instance of Example 1. After the feces sampling implement is accommodated in the container, it is fitted together with the funnel-shaped member, whereby the feces sampling implement cannot be pulled out or it becomes difficult for the feces sampling implement to be pulled out. This is an example where a part of the feces sampling implement is recessed so that it is fitted together with the funnel-shaped member of the feces sampling container to prevent the feces sampling implement from being reversely moved.

### Experimental Example 3

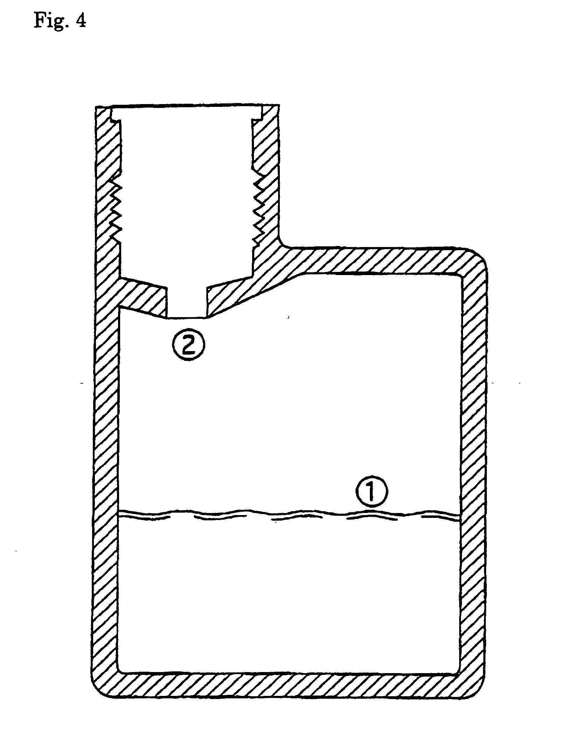

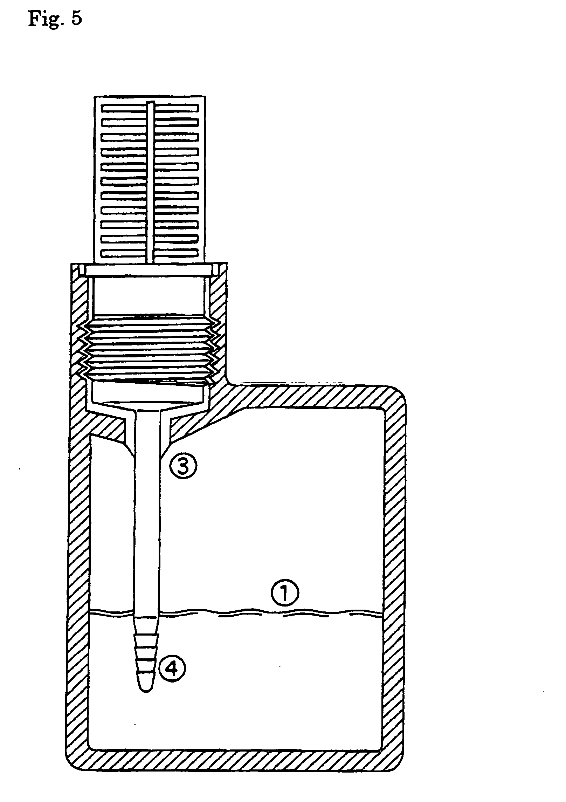

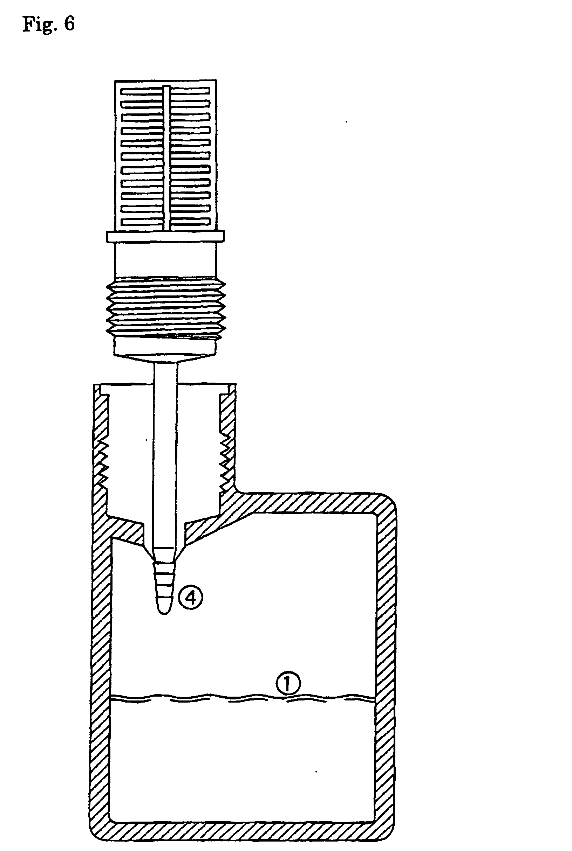

Fig. 4 is an example of a feces sampling container where the insertion slot for the feces sampling implement is closed off by a thin film. By inserting the feces sampling implement through the insertion slot of the feces sampling container having the thin film, the thin film is broken so that the thin film faces inward as it is retained in the container. Fig. 5 shows a state where the feces sampling implement is accommodated in the body of the feces sampling container. Fig. 6 shows a state where the accommodated feces sampling implement is being pulled out from the feces sampling container. The thin film facing inward catches on convex portions and/or concave portions disposed at a tip end of the feces sampling implement, whereby it becomes difficult for the feces sampling implement to be pulled out and multiple feces sampling can be prevented.

### Industrial Applicability

As described above, the feces sampling implement, the feces sampling container and the feces sampling device of the invention are designed so that, once the feces sampling implement has been inserted and accommodated inside the feces sampling container, it becomes impossible or difficult for the feces sampling implement to again be pulled out from the feces sampling container, whereby multiple sample collection is prevented and it becomes possible to collect a specific amount of feces.

## Claims

1. In a feces sampling device including a feces sampling implement and a feces sampling container that can accommodate the feces sampling implement, a feces sampling implement comprising a measure that can control further taking out and putting in of the feces sampling implement once the feces sampling implement has been inserted and accommodated inside the feces sampling container.

2. The feces sampling implement of claim 1, wherein a reverse-motion or pullout prevention function measure is effected after the feces sampling implement has been inserted and accommodated in the feces sampling container.

3. The feces sampling implement of claim 2, wherein the reverse-motion or pullout prevention function is retained by the feces sampling implement including convex portions and/or concave portions at a part of its structure.

4. A feces sampling device retaining a reverse-motion or pullout prevention function by combining a container that can accommodate the feces sampling implement of at least any one of claims 1 to 3 and the feces sampling implement.

5. The feces sampling device of claim 4, wherein a funnel-shaped member is disposed at a part of the feces sampling container that can accommodate the feces sampling implement.

6. The feces sampling device of claim 4 or 5, wherein a thin film is disposed at an insertion slot for the feces sampling implement in the feces sampling container that can accommodate the feces sampling implement.

7. A feces sampling method using the feces sampling implement or the feces sampling device of at least any one of claims 1 to 6.

8. A method for preparing a sample of occult blood in feces by the feces sampling method of claim 7.

9. Amethod of testing for occult blood in feces using the sample obtained in claim 8.
